(19) Europäisches Patentamt
European Patent Office
Office européen des brevets

(11) **EP 2 533 827 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**21.12.2022 Patentblatt 2022/51**

(45) Hinweis auf die Patenterteilung:
**09.09.2015 Patentblatt 2015/37**

(21) Anmeldenummer: **11704926.2**

(22) Anmeldetag: **07.02.2011**

(51) Internationale Patentklassifikation (IPC):
**A61M 1/36** (2006.01)

(52) Gemeinsame Patentklassifikation (CPC):
**A61M 1/3653; A61M 1/361; A61M 1/3655; A61M 1/3656; A61M 1/3658; A61M 1/3659;** A61M 2205/18; A61M 2230/201; A61M 2230/205; A61M 2230/207; A61M 2230/208

(86) Internationale Anmeldenummer:
**PCT/EP2011/000560**

(87) Internationale Veröffentlichungsnummer:
**WO 2011/098243 (18.08.2011 Gazette 2011/33)**

(54) **VORRICHTUNG UND VERFAHREN ZUR ÜBERWACHUNG EINES GEFÄSSZUGANGS FÜR EINE EXTRAKORPORALE BLUTBEHANDLUNG**

DEVICE AND METHOD FOR MONITORING A VASCULAR ACCESS FOR AN EXTRACORPOREAL BLOOD TREATMENT

DISPOSITIF ET PROCÉDÉ POUR SURVEILLER UN ACCÈS VASCULAIRE POUR UN TRAITEMENT SANGUIN EXTRA-CORPOREL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.02.2010 DE 102010007914**

(43) Veröffentlichungstag der Anmeldung:
**19.12.2012 Patentblatt 2012/51**

(73) Patentinhaber: **Fresenius Medical Care Deutschland GmbH**
**61352 Bad Homburg v.d.H. (DE)**

(72) Erfinder: **ZHANG, Wei**
**97464 Niederwerrn (DE)**

(74) Vertreter: **Oppermann, Frank**
**OANDO Oppermann & Oppermann LLP**
**Wilhelminenstrasse 1a**
**65193 Wiesbaden (DE)**

(56) Entgegenhaltungen:
WO-A1-97/10013          WO-A1-2008/000433
DE-A1-102006 032 815    US-A1- 2002 062 098
US-A1- 2003 128 125     US-A1- 2009 082 653
US-A1- 2009 088 683     US-A1- 2009 221 948

EP 2 533 827 B2

## Beschreibung

Die Erfindung betrifft eine Vorrichtung zur extrakorporalen Blutbehandlung mit einem extrakorporalen Blutkreislauf.

Auf dem Gebiet der Medizintechnik sind verschiedene extrakorporale Blutbehandlungsvorrichtungen bekannt, die über einen extrakorporalen Blutkreislauf verfügen. Zu den bekannten extrakorporalen Blutbehandlungsvorrichtungen zählen beispielsweise Dialysevorrichtungen, die einen Zugang zu dem Gefäßsystem des Patienten erforderlich machen. Bei der extrakorporalen Blutbehandlung wird dem Patienten über eine arterielle Schlauchleitung mit einer arteriellen Punktionskanüle Blut entnommen, das dem Patienten über eine venöse Schlauchleitung mit einer venösen Punktionskanüle wieder zugeführt wird. Zum Fördern des Bluts im extrakorporalen Blutkreislauf verfügen die extrakorporalen Blutbehandlungsvorrichtungen über eine Blutpumpe.

Bei der extrakorporalen Blutbehandlung besteht trotz regelmäßiger Überwachung des Gefäßzugangs durch das Krankenhauspersonal grundsätzlich die Gefahr, dass die Punktionskanülen unbemerkt aus dem Blutgefäß des Patienten herausrutschen. Während ein Herausrutschen der arteriellen Kanüle mit einem Ansaugen von Luft in die arterielle Schlauchleitung verbunden ist, führt das Herausrutschen der venösen Kanüle zu dem gefürchteten Freifluss des Blutes in die Umgebung. Wenn das Herausrutschen der venösen Kanüle daher nicht sofort erkannt wird, besteht die Gefahr, dass der Patient verblutet.

Zur Überwachung des Gefäßzugangs sind verschiedene Vorrichtungen unterschiedlicher Ausbildung bekannt. Die bekannten Überwachungsvorrichtungen greifen im Allgemeinen auf die standardmäßig in den Blutbehandlungsvorrichtungen vorhandenen Sicherheitsvorrichtungen zurück, die bei einem nicht ordnungsgemäßen Gefäßzugang eine sofortige Unterbrechung des Blutflusses im extrakorporalen Blutkreislauf auslösen.

Aus der WO 99/29356 A1 ist eine Überwachungsvorrichtung für einen Gefäßzugang bekannt, bei der die Stärke eines elektrischen Stroms gemessen wird, der durch die Flüssigkeit in der Schlauchleitung fließt. Die US 2004/0254513 A1 beschreibt eine Überwachungsvorrichtung, bei der die Impedanz zwischen zwei an der arteriellen und venösen Schlauchleitung angeordneten Elektroden gemessen wird. Nachteilig ist, dass die bekannten Vorrichtungen die Schaffung einer elektrischen Verbindung zu der in den Schlauchleitungen strömenden Flüssigkeit erfordern.

Aus der WO 2006/008866A1 und US 2005/0038325 A1 sind Überwachungsvorrichtungen bekannt, die den Austritt von Blut an der Punktionsstelle detektieren können. Diese Vorrichtungen verfügen über einen Feuchtigkeitssensor.

Zur Bestimmung der Konzentration von bestimmten Bestandteilen im Blut eines Patienten, beispielsweise zur Bestimmung der Konzentration von Hämoglobin im Blut oder des Hämatokrit, sind verschiedene Verfahren bekannt. Als Stand der Technik sind Verfahren zur Messung der Konzentration von Blutbestandteilen bekannt, die eine Entnahme einer Blutprobe voraussetzen. Es sind aber auch Messverfahren bekannt, bei denen die Konzentration von Bestandteilen im Blut nicht invasiv gemessen wird, das in einer Schlauchleitung strömt. Diese Verfahren finden insbesondere dann Anwendung, wenn bei einer extrakorporalen Blutbehandlung das Blut durch die Schlauchleitung eines extrakorporalen Blutkreislaufs fließt.

Die WO 2008/000433 A1 beschreibt ein Verfahren und eine Vorrichtung zur Bestimmung der Konzentration von Blutbestandteilen in einer mit Blut gefüllten transparenten Schlauchleitung eines extrakorporalen Blutkreislaufs einer extrakorporalen Blutbehandlungsvorrichtung. Das bekannte Verfahren und die bekannte Vorrichtung erlauben insbesondere die Bestimmung der Hämoglobinkonzentration und des Anteils der roten Blutkörperchen (Erythrozyten) am Gesamtvolumen des Bluts. Während der Messung ist die Schlauchleitung zwischen zwei parallelen Anlageflächen eingespannt. Die Messung der Hämoglobinkonzentration oder des Hämatokrit beruht auf der Streuung oder Transmission von elektromagnetischer Strahlung im Blut. Mit einem Lichtemitter wird Licht einer bestimmten Wellenlänge durch die transparente Schlauchleitung in das Blut eingekoppelt, während mit einem Lichtdetektor das gestreute oder transmittierte Licht gemessen wird. Der Hämatokrit wird dann aus dem Verhältnis der Intensität des in das Blut eintretenden und aus dem Blut austretenden Lichts ermittelt.

Bei extrakorporalen Blutbehandlungsverfahren, beispielsweise bei der Hämodialyse, Hämofiltration oder Hämodiafiltration wird als Zugang zum Blutgefäßsystem des Patienten häufig operativ eine arteriovenöse Fistel angelegt. Ebenso ist der Einsatz eines Implantats möglich. Wenn nachfolgend von einer "Fistel" die Rede ist, wird darunter jede Art der Verbindung zwischen einer Vene und einer Arterie zur Schaffung eines Gefäßzugangs verstanden.

Im dialysefreien Zeitraum entspricht der Blutfluss in der Fistel einem funktionellen Links/Rechts-Shunt, bei dem ein Anteil des arteriellen Bluts aus dem Herzminutenvolumen (HMV) unter Umgehung einer peripheren Nutzung direkt dem venösen System und dem Herzen zugeführt wird. Der Fistelfluss rezirkuliert über Herz und Lungen. Der fraktionelle Anteil des Fistelflusses am Herzminutenvolumen wird als kardiopulmonare Rezirkulation definiert. Während der Dialysebehandlung strömt das vom linken Ventrikel des Herzens emittierte Blut zum größten Teil in die Kapillarsysteme aller Organe und zu einem kleinen Teil in die Fistel. Für den Fall, dass der Blutfluss im extrakorporalen Blutkreislauf kleiner als der Fluss des in die Fistel bzw. aus der Fistel fließenden Bluts ist, fließt das Fistelblut zum einen Teil durch den extrakorporalen Blutkreislauf und zum anderen Teil durch die Fistel. Das extrakorporale Blut, das durch die Fistel fließende Blut und das aus den Kapillarsystemen stammende Blut vereinigen sich schließlich wieder im Rücklauf zum Herzen. Wenn der extrakorporale Blutfluss hingegen größer als der Fistelfluss ist, rezirkuliert Blut aus dem extrakorporalen Blutkreislauf, wobei die Fistel vom venösen zum arteriellen Anschluss durchflossen wird.

Aus der WO 2009/065611 A1 ist ein Verfahren und eine Vorrichtung zum Bestimmen der Rezirkulation in einer Fistel oder der kardiopulmonalen Rezirkulation bekannt. Das bekannte Verfahren und die bekannte Vorrichtung beruhen darauf, dass die Summe von der Fistelrezirkulation ($R_A$) und dem kardiopulmonalen Rezirkulationsanteil ($R_{CP}$), d.h. die Gesamtrezirkulation ($R$), für zwei unterschiedliche Blutflussraten bestimmt wird. Die WO 2009/065611 A1 beschreibt auch den theoretischen Hintergrund zu dem Effekt der Fistelrezirkulation und kardiopulmonalen Rezirkulation. Diese Effekte sind auch in dem Fachartikel "Automatische Messung der Rezirkulation" von Krämer und Polaschegg, EDTNA-ERCA Journal Vol. XIX, Nr. 3 (1993) beschrieben.

US 2002/0062098 offenbart ein Verfahren und eine Vorrichtung zum Bestimmen der Rezirkulation, wobei die Hämoglobinkonzentration des in der arteriellen Leitung fließenden Bluts gemessen wird.

Der Erfindung liegt die Aufgabe zugrunde, den Zugang zu dem Gefäß eines Patienten ohne umfangreiche Veränderungen an der Blutbehandlungsvorrichtung und ohne die Verwendung separater Komponenten mit hoher Zuverlässigkeit zu überwachen.

Die Lösung dieser Aufgabe erfolgt erfindungsgemäß mit den Merkmalen des Anspruchs 1. Vorteilhafte Ausführungsformen der Erfindung sind Gegenstand der Unteransprüche.

Die erfindungsgemäße Vorrichtung beruht auf der Überwachung einer charakteristischen Eigenschaft des in der arteriellen Blutleitung des extrakorporalen Blutkreislaufs strömenden Bluts, wobei die charakteristische Eigenschaft die Konzentration von Hämoglobin in dem im extrakorporalen Blutkreislauf strömenden Blut ist.

Bei einem nicht ordnungsgemäßen Gefäßzugang verändern sich die Strömungsverhältnisse im kommunizierenden intra- und extrakorporalen Blutzirkulationssystem. Diese Veränderungen der Strömungsverhältnisse sind als Änderung eines Blutparameters nachweisbar. Es ist ausreichend, wenn nur ein einziger Blutparameter überwacht wird. Zur Erhöhung der Zuverlässigkeit der erfindungsgemäßen Messung können aber auch mehrere Blutparameter überwacht werden, wobei dann bei einer Veränderung sämtlicher überwachter Blutparameter auf einen nicht ordnungemäßen Gefäßzugang geschlossen wird.

Für die erfindungsgemäße Vorrichtung ist entscheidend, dass die Messung der charakteristischen Eigenschaft des Bluts in oder an der arteriellen Blutleitung; d.h. zwischen Dialysator und arterieller Kanüle erfolgt.

Bei einem Patienten ohne Fistel durchströmt das gesamte Blut den Weg über Herz und Lunge sowie sämtliche Kapillarsysteme der inneren Organe, der Muskulatur und Haut etc., um dann wieder zurück zum Herzen zu strömen. Bei einem Dialysepatienten mit Fistel strömt ein Teil des zirkulierenden Bluts nicht mehr durch die Kapillarsysteme, sondern umgeht diese und nimmt den Weg über die parallel zu den Kapillarsystemen angelegte Fistel. Diese so genannte "kardiopulmonale Rezirkulation" ist bei einem Patienten mit Fistel stets vorhanden, und zwar sowohl während einer Dialysebehandlung als auch wenn der Patient nicht an einen extrakorporalen Blutkreislauf angeschlossen ist. Dementsprechend wird bei angeschlossenem extrakorporalem Blutkreislauf ein Teil des im Dialysator behandelten und über die venöse Nadel in die Fistel zurückgegebenen Bluts unter Umgehung der Kapillarsysteme direkt über Herz und Lunge zurück in die Fistel geleitet. Diese kardiopulmonale Rezirkulation ist stets vorhanden und unvermeidbar. Unabhängig von der kardiopulmonalen Rezirkulation kann in der Fistel beim möglichen Auftreten einer Fistelrezirkulation ein Teil des behandelten, über die venöse Kanüle in die Fistel zurückgeführten Bluts auch auf diesem direkten Weg erneut in die arterielle Kanüle eintreten.

Beide Rezirkulationsvorgänge wirken sich auf die Messwerte verschiedener Blutparameter in der arteriellen Leitung aus. Beim Herausziehen der venösen Nadel aus der Fistel gelangt das in der venösen Blutleitung strömende frisch behandelte Blut schlagartig nicht mehr in Fistel. Folglich entfällt zum einen die Beimischung von bereits behandeltem Blut über den Kurzschlussweg der möglicherweise vorhandenen Fistelrezirkulation in die arterielle Leitung und zum anderen entfällt dabei stets die Beimischung von frisch behandelten Blut über den Umweg der kardiopulmonalen Rezirkulation in die arterielle Leitung.

Stromabwärts nach dem Dialysator, also in der venösen Leitung, sind während der Dialyse beispielsweise die Hämoglobinkonzentration, die Hämatokritkonzentration und die Viskosität im Vergleich zu den entsprechenden Messwerten in der arteriellen Leitung aufgrund des Wasserentzugs im Dialysator erhöht, d.h. es erfolgt im Dialysator eine Aufkonzentrierung. Im Falle einer venösen Nadeldiskonnektion führt der Wegfall der Beimischung von höher konzentriertem Blut in die Fistel auf der arteriellen Seite zu einer Verringerung der entsprechenden Messwerte.

Es hat sich gezeigt, dass bei einer venösen Nadeldiskonnektion die Beimischung von im Dialysator bereits behandeltem Blut über die Wege der kardiopulmonalen Rezirkulation und der möglicherweise vorliegenden Fistelrezirkulation in die arterielle Blutleitung wegfällt. Daher kann bei einer venösen Nadeldiskonnektion die Veränderung verschiedener Blutparameter in der arteriellen Blutleitung des extrakorporalen Blutkreislaufs nachgewiesen werden. Da die kardiopulmonale Rezirkulation immer vorhanden ist, ist selbst dann eine Überwachung des Patientenzugangs möglich, wenn eine Fistelrezirkulation nicht gegeben ist.

Grundsätzlich können alle Blutparameter als Messgröße herangezogen werden, die entweder zu einer nachweisbaren Erhöhung oder Verringerung des Parameters in der arteriellen Blutleitung führen. Bei dem Blutparameter handelt es sich um die Hämoglobinkonzentration.

Auf eine venöse Nadeldiskonnektion kann geschlossen werden, falls eine Verringerung des entsprechenden Messwerts eines überwachten Blutparameters in der arteriellen Blutleitung des extrakorporalen Blutkreislaufs gemessen wird, wobei der korrespondierende Wert des gleichen Blutparameters an einer Stelle nach dem Dialysator unter Zugrundelegung eines fehlerfreien Gefäßzugangs größer ist als vor dem Dialysator.

Analog kann auf eine venöse Nadeldiskonnektion geschlossen werden, falls eine Vergrößerung des entsprechenden Messwerts eines überwachten Blutparameters in der arteriellen Blutleitung des extrakorporalen Blutkreislaufs gemessen wird, wobei der korrespondierende Wert des gleichen Blutparameters an einer Stelle nach dem Dialysator unter Zugrundelegung eines fehlerfreien Gefäßzugangs kleiner ist als vor dem Dialysator.

Demzufolge sind solche Blutparameter für die erfindungsgemäße Überwachung einer venösen Nadeldiskonnektion geeignet, deren Messwerte vor bzw. nach dem Dialysator unterschiedliche Beträge aufweisen.

Zu einer Änderung des Blutparameters im Dialysator kann es beispielsweise durch eine "Eindickung" des Bluts infolge eines Wasserentzugs im Dialysator aufgrund einer Ultrafiltration kommen. Aber auch der vorhandene Wärmeübergang zwischen der Blut- und Dialysatseite kann zu einer Änderung des Parameters führen.

Das Verhalten beispielsweise der Bluttemperatur, die aber von der Erfindung nicht umfasst ist, als möglichem Blutparameter im extrakorporalen Blutkreislauf hängt vom Beheizungsregime auf der Dialysatseite ab. Für den Fall, dass dem Blut im Dialysator geringfügig Wärme entzogen wird, liegt die Bluttemperatur bei fehlerfreiem Gefäßzugang stromabwärts des Dialysators geringfügig unter der entsprechenden Temperatur auf der arteriellen Seite. Es wird dementsprechend über die Wege der kardiopulmonalen Rezirkulation und der Fistelrezirkulation kühleres Blut in der Fistel beigemischt und in die arterielle Leitung geleitet. Im Falle einer venösen Nadeldiskonnektion entfällt die Beimischung von kühlerem Blut in die arterielle Leitung, was dort zu einer messbaren Temperaturerhöhung führt. Für den umgekehrten Fall, dass dem Blut im Dialysator geringfügig Wärme zugeführt wird, kann eine venöse Nadeldiskonnektion an einem Abfall der Bluttemperatur in der arteriellen Leitung erkannt werden, weil dann die Beimischung von wärmerem Blut in die arterielle Leitung entfällt.

Die Messung der Hämoglobinkonzentration hat den Vorteil, dass die im Stand der Technik bekannten Verfahren Verwendung finden können, mit denen sich die Hämoglobinkonzentration schnell bestimmen lässt. Grundsätzlich können sämtliche Messverfahren zur Bestimmung der Hämoglobinkonzentration eingesetzt werden. Vorteilhafterweise werden die bekannten optischen Verfahren eingesetzt. Die Hämoglobinkonzentration kann aber auch mittels der bekannten Ultraschall-Messverfahren bestimmt werden. Von Vorteil ist, wenn die Messung der Hämoglobinkonzentration nicht invasiv erfolgt.

Die erfindungsgemäße Vorrichtung verfügt über Mittel zum Messen der Hämoglobinkonzentration in dem in der arteriellen Blutleitung des extrakorporalen Blutkreislaufs strömenden Blut und eine Steuer- und Recheneinheit, die derart ausgebildet ist, dass bei einer Verringerung der Hämoglobinkonzentration um einen Betrag, der einen vorbestimmten Betrag überschreitet, auf einen nicht ordnungsgemäßen Gefäßzugang geschlossen wird. Die Verringerung der Hämoglobinkonzentration um einen Betrag, der einen vorbestimmten Betrag überschreitet, kann mit unterschiedlichen mathematischen Auswertverfahren festgestellt werden.

Bei einer bevorzugten Ausführungsform der Erfindung weist die Steuer- und Recheneinheit Mittel zum Vergleichen der gemessenen Hämoglobinkonzentration mit einem vorgegebenen Grenzwert und Mittel zum Erzeugen eines Steuersignals auf, wenn der Betrag der Differenz zwischen der gemessenen Hämoglobinkonzentration und dem vorgegebenen Grenzwert größer als Null ist.

Bei einer alternativen bevorzugten Ausführungsform weist die Steuer- und Recheneinheit Mittel zum Vergleichen der zu einem ersten vorausgehenden Zeitpunkt gemessenen ersten Hämoglobinkonzentration mit einer zu einem zweiten nachfolgenden Zeitpunkt gemessenen zweiten Hämoglobinkonzentration auf. Das Steuersignal wird erzeugt, wenn der Betrag der Differenz zwischen der ersten und zweiten Hämoglobinkonzentration größer als ein vorgegebener Grenzwert ist. Von Vorteil ist, dass auch bei einer Änderung der Hämoglobinkonzentration aufgrund anderer Umstände die Verringerung der Hämoglobinkonzentration in Folge einer Nadeldiskonnektion mit hoher Sicherheit erkannt werden kann, wenn das Zeitintervall zwischen den beiden Messungen so kurz gewählt wird, dass innerhalb des Zeitintervalls eine Änderung der Hämoglobinkonzentration aufgrund anderer Umstände nicht zu erwarten ist. Beispielweise sollten die Messungen zu zwei Zeitpunkten erfolgen, zwischen denen die Blutflussrate und/oder Ultrafiltrationsrate nicht verändert wird. Die Messungen zu den beiden aufeinander folgenden Zeitpunkten erfolgen vorzugsweise während der gesamten Blutbehandlung.

Zur Erhöhung der Zuverlässigkeit des erfindungsgemäßen Messverfahrens kann die kardiopulmonale Rezirkulation gemessen und mit einem vorgegebenen oberen und unteren Grenzwert verglichen werden, wobei dann bei einer Verringerung der Hämoglobinkonzentration um einen Betrag, der einen vorbestimmten Betrag überschreitet, auf einen nicht ordnungsgemäßen Gefäßzugang geschlossen wird, wenn die gemessene kardiopulmonale Rezirkulation zwischen dem vorgegebenen oberen und unteren Grenzwert liegt. Anstelle eines Wertebereichs kann aber auch nur ein oberer oder unterer Grenzwert für den Vergleich der gemessenen kardiopulmonalen Rezirkulation festgelegt werden.

Eine weitere bevorzugte Ausführungsform der erfindungsgemäßen Vorrichtung sieht eine Alarmeinheit vor, die einen akustischen und/oder optischen und/oder taktilen Alarm gibt, wenn die Steuer- und Recheneinheit einen nicht ordnungsgemäßen Gefäßzugang feststellt.

Die erfindungsgemäße Vorrichtung zur extrakorporalen Blutbehandlung verfügt über eine Vorrichtung zur Überwachung des Gefäßzugangs. Von Vorteil ist, dass die erfindungsgemäße Vorrichtung nicht von externen Komponenten Gebrauch machen, die zusätzliche Handgriffe erfordern oder die Bewegungsfreiheit des Patienten unnötig einschränken.

Weiterhin ist vorgesehen, dass die Steuereinheit der Blutbehandlungsvorrichtung einen Eingriff in die Maschinensteuerung vornimmt, wenn die Steuer- und Recheneinheit der Überwachungsvorrichtung bei einem nicht ordnungsgemäßen Gefäßzugang ein Steuersignal erzeugt. Die Steuereinheit ist derart ausgebildet, dass als Eingriff in die Maschinensteuerung die im extrakorporalen Blutkreislauf angeordnete Blutpumpe angehalten wird und/oder ein in der venösen Blutleitung angeordnetes Absperrorgan geschlossen wird. Dadurch wird wirkungsvoll verhindert, dass bei einem nicht ordnungsgemäßen Gefäßzugang, beispielsweise wenn die venöse Punktionskanüle herausgerutscht ist oder eine Leckage im Schlauchsystem vorliegt, Blut in die Umgebung gelangt.

Im Folgenden wird ein Ausführungsbeispiel der Erfindung unter Bezugnahme auf die Figuren näher erläutert.

Es zeigen:

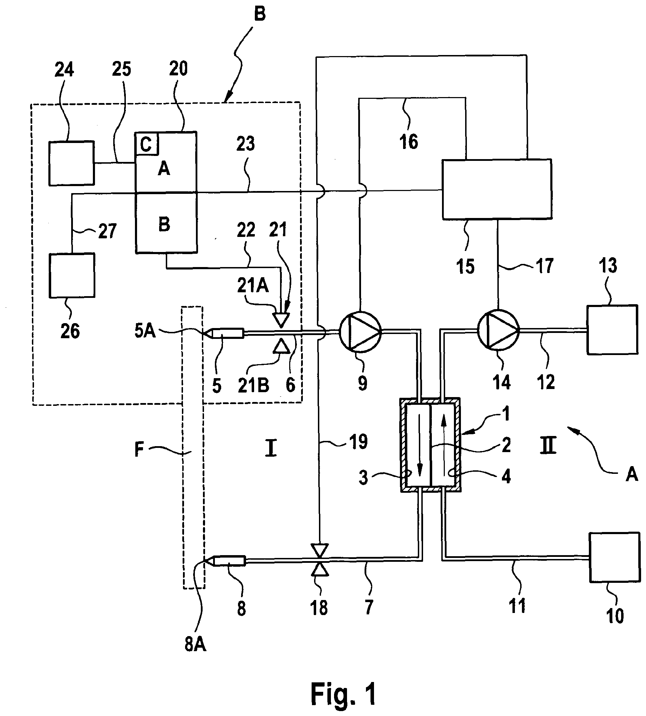

Fig. 1    in vereinfachter schematischer Darstellung die wesentlichen Komponenten einer erfindungsgemäßen extrakorporalen Blutbehandlungsvorrichtung mit einer Vorrichtung zur Überwachung eines Gefäßzugangs und

Fig. 2    die Änderung der Hämoglobinkonzentration in Abhängigkeit von der Ultrafiltrationsrate.

Die erfindungsgemäße Vorrichtung kann eine separate Einheit bilden oder auch Bestandteil der extrakorporalen Blutbehandlungsvorrichtung sein. Wenn die erfindungsgemäße Vorrichtung Bestandteil der Blutbehandlungsvorrichtung ist, kann die erfindungsgemäße Vorrichtung von bestimmten Baugruppen oder Bauteilen Gebrauch machen, die in der Blutbehandlungsvorrichtung ohnehin vorhanden sind.

Nachfolgend wird eine extrakorporale Blutbehandlungsvorrichtung A beschrieben, die über eine Vorrichtung B zur Überwachung des Gefäßzugangs verfügt. Die Figur zeigt nur die wesentlichen Komponenten der Blutbehandlungsvorrichtung in schematischer Darstellung, da Blutbehandlungsvorrichtungen, beispielsweise Hämodialysevorrichtungen, Hämofiltrationsvorrichtungen oder Hämodiafiltrationsvorrichtungen, als solche dem Fachmann bekannt sind.

Bei der Blutbehandlungsvorrichtung handelt es sich um eine bekannte Hämodialysevorrichtung, die einen Dialysator 1 aufweist, der durch eine semipermeable Membran 2 in eine Blutkammer 3 und eine Dialysierflüssigkeitskammer 4 unterteilt ist. An dem arteriellen Teil 5A einer Fistel F des Patienten ist mit einer arteriellen Punktionskanüle 5 eine arterielle Schlauchleitung 6 angeschlossen, die zu dem Einlass der Blutkammer 3 des Dialysators führt. Von dem Auslass der Blutkammer 3 des Dialysators geht eine venöse Schlauchleitung 7 ab, die mit einer venösen Punktionskanüle 8 an dem venösen Teil 8A der Fistel F angeschlossen ist. Das Blut wird im extrakorporalen Blutkreislauf I mit einer Blutpumpe 9 gefördert, die an der arteriellen Schlauchleitung 6 vorgesehen ist.

Der Dialysierflüssigkeitskreislauf II der Hämodialysevorrichtung umfasst eine Dialysierflüssigkeitsquelle 10, an der die Dialysierflüssigkeitszuführleitung 11 angeschlossen ist, die zu dem Einlass der Dialysierflüssigkeitskammer 4 des Dialysators führt. Von dem Auslass der Dialysierflüssigkeitskammer 4 des Dialysators geht eine Dialysierflüssigkeitsabführleitung 12 ab, die zu einem Auslass 13 führt. Die Dialysierflüssigkeit wird im Dialysierflüssigkeitskreislauf II mit einer Dialysierflüssigkeitspumpe 14 gefördert, die an der Dialysierflüssigkeitsabführleitung 12 vorgesehen ist.

Die Steuerung der Dialysevorrichtung übernimmt eine zentrale Steuereinheit 15, die über Steuerleitungen 16, 17 die Blut- und die Dialysierflüssigkeitspumpe 9, 14 ansteuert. Mit der Blutpumpe 9 wird die Blutflussrate $Q_B$ eingestellt.

Stromab der Blutkammer 3 des Dialysators 1 befindet sich an der venösen Schlauchleitung 7 eine elektromagnetisch betätigbare Schlauchklemme 18, die über eine weitere Steuerleitung 19 von der zentralen Steuereinheit 15 geöffnet bzw. geschlossen werden kann. Die Flüssigkeitsströmung ist im extrakorporalen Blutkreislauf I unterbrochen, wenn die venöse Schlauchklemme 18 geschlossen ist, sodass Blut nicht in die Umgebung gelangen kann.

Neben den in der Figur gezeigten Komponenten umfasst die Dialysevorrichtung noch weitere Baugruppen, die aber der Übersichtlichkeit halber nicht dargestellt sind. Zu diesen zählen beispielsweise eine Bilanziereinrichtung zum Bilanzieren von frischer gegen verbrauchte Dialysierflüssigkeit und eine Ultrafiltrationseinrichtung, um dem Patienten mit einer vorgegebenen Ultrafiltrationsrate $Q_{UF}$ Flüssigkeit entziehen zu können.

Die Vorrichtung B zur Überwachung des venösen Gefäßzugangs verfügt über eine Steuer-und Recheneinheit 20, die in der Figur als separate Einheit dargestellt ist. Die Steuer- und Recheneinheit 20 kann aber auch Bestandteil der zentralen Steuereinheit 15 der Blutbehandlungsvorrichtung sein.

Darüber hinaus verfügt die Überwachungsvorrichtung B über Mittel zum Messen der Konzentration von Hämoglobin in dem in der arteriellen Blutleitung des extrakorporalen Blutkreislaufs I strömenden Blut.

Die Mittel zum Messen der Hämoglobinkonzentration HB weisen eine nicht invasive optische Messeinheit 21 auf, die an der arteriellen Blutleitung 6 stromab der arteriellen Punktionskanüle 5 und stromauf der Blutpumpe 9 angeordnet ist. Die Messwerte der Messeinheit 21 empfängt die Steuer- und Recheneinheit 20 über eine Datenleitung 22. Vorrichtungen zur nicht invasiven optischen Messung der Hämoglobinkonzentration sind dem Fachmann bekannt. Anstelle optischer Messvorrichtungen können aber auch die bekannten Vorrichtungen zur Bestimmung der Hämoglobinkonzentration auf der Grundlage einer Ultraschall-Messung verwendet werden. Für die Erfindung ist unerheblich, wie die Hämoglobinkonzentration gemessen wird.

Die WO 2008/000433 A1 beispielsweise beschreibt eine bekannte Vorrichtung zur Messung der Hämoglobinkonzentration, die über eine Messeinheit 21 verfügt, die einen Lichtemitter 21 A und einen Lichtdetektor 21B aufweist, um Licht mit einer vorgegebenen Wellenlänge durch die arterielle Blutleitung 6, bei der es sich um eine für Licht, beispielsweise Infrarotlicht, durchlässige Schlauchleitung handelt, in das Blut einkoppeln und auskoppeln zu können. Aus dem Verhältnis der Intensität des ein- und ausgekoppelten Lichts wird die Hämoglobinkonzentration ermittelt. Die Zuordnung zwischen dem Verhältnis der Intensität des ein- und ausgekoppelten Lichts und der Hämoglobinkonzentration kann in einem Speicher C der Rechen- und Auswerteinheit 20 hinterlegt sein.

Zur Messung der Hämoglobinkonzentration kann auch der optische Blutvolumenmonitor (OBVM) verwendet werden, der aus der EP 1 748 292 A1 bekannt ist.

Die Steuer- und Recheneinheit 20 der Überwachungsvorrichtung weist bei einem ersten Ausführungsbeispiel Mittel 20A zum Vergleichen der gemessenen Hämoglobinkonzentration mit einem vorgegebenen Grenzwert auf. Darüber hinaus weist die Steuer- und Recheneinheit 20 Mittel 20B zum Erzeugen eines Steuersignals auf, das die zentrale Steuereinheit 15 über eine Datenleitung 23 empfängt.

Für die extrakorporale Blutbehandlung werden eine bestimmte Blutflussrate $Q_B$ und eine bestimmte Ultrafiltrationsrate $Q_{UF}$ vom behandelnden Arzt vorgegeben. Die zentrale Steuereinheit 15 der Blutbehandlungsvorrichtung stellt die Drehzahl n der Blutpumpe 9 derart ein, dass das Blut im extrakorporalen Blutkreislauf I mit der vorgegebenen Flussrate $Q_B$ gefördert wird. Die nicht dargestellte Ultrafiltrationseinrichtung stellt sicher, dass dem Patienten Flüssigkeit mit der vorgegebenen Ultrafiltrationsrate $Q_{UF}$ entzogen wird.

Während der extrakorporalen Blutbehandlung wird die Hämoglobinkonzentration HB laufend überwacht. Die gemessene Hämoglobinkonzentration wird laufend mit dem vorgegebenen Grenzwert verglichen, um eine Verringerung der Hämoglobinkonzentration feststellen zu können. Wenn der Betrag der Differenz zwischen der gemessenen Hämoglobinkonzentration und dem vorgegebenen Grenzwert größer als Null ist, stellt die Steuer- und Recheneinheit 20 fest, dass ein nicht ordnungsgemäßer Gefäßzugang vorliegt, d.h. die venöse Punktionskanüle herausgerutscht ist. Der vorgegebene Grenzwert ist abhängig von der Blutflussrate $Q_B$ und der Ultrafiltrationsrate $Q_{UF}$. In dem Speicher C der Steuer- und Recheneinheit können daher verschiedene Grenzwerte hinterlegt sein, die verschiedenen Blutflussraten und Ultrafiltrationsraten zugeordnet sind, sodass die Steuer-und Recheneinheit 20 den passenden Grenzwert für die momentane Blutflussrate $Q_B$ und Ultrafiltrationsrate $Q_{UF}$ auswählen kann.

Bei dem Grenzwert für die Hämoglobinkonzentration oder auch eines anderen Blutparameters kann es sich um einen Grenzwert handeln, der vor Beginn oder zu Beginn der Behandlung fest vorgegeben wird. Es kann sich aber auch um einen dynamischen Grenzwert handeln, der in Abhängigkeit vom Blutfluss $Q_B$ und der Ultrafiltrationsrate $Q_{UF}$ während der Behandlung fortlaufend oder in bestimmten Zeitabständen ermittelt und aktualisiert wird. Erfindungsgemäß erfolgt dann der Vergleich des Messwerts des Blutparameters mit einem aktualisierten Grenzwert. Die Zuverlässigkeit der Auswertung kann damit weiter verbessert werden. Für das Ausführungsbeispiel mit der Hämoglobinkonzentration als Blutparameter ergibt sich dann die folgende Gleichung zur Berechnung und Aktualisierung des vorgegebenen Grenzwerts *Thresh* für die Hämoglobinkonzentration:

$$Thresh = k \cdot HB \cdot \frac{\alpha \cdot (R_A + R_{CP})}{1 - \alpha - (R_A + R_{CP})} = k \cdot HB \cdot \frac{\alpha \cdot R_{BTM}}{1 - \alpha - R_{BTM}} \qquad (1 > k > 0, z.B. \, k = 0{,}5)$$

Dabei ist $R_A$ die Rezirkualtion in der Fistel und $R_{CP}$ der kardiopulmonäre Rezirkulationsanteil im extrakorporalen Blutkreislauf. $R_{BTM}$ ist die während der Dialyse gemessene Gesamtrezirkualtion. Die Gesamtrezikulation kann mit einer dem Fachmann bekannten Messeinrichtung gemessen werden. Eine derartige Messeinrichtung ist beispielsweise aus der WO 2009/065611 A1 bekannt. In der Figur ist diese Messeinrichtung 26, die Bestandteil der bekannten Dialysevorrichtungen ist, nur andeutungsweise dargestellt. Die Messeinrichtung 26 ist mit der Steuer-und Recheneinheit 26 über eine Datenleitung 27 verbunden.

Die Überwachungsvorrichtung B weist eine Alarmeinheit 24 auf, die das Steuersignal der Steuer- und Recheneinheit 20 über eine Datenleitung 25 empfängt. Die Alarmeinheit 24 gibt dann einen akustischen und/oder optischen und/oder taktilen Alarm. Die Alarmeinheit kann aber auch Bestandteil der Blutbehandlungsvorrichtung sein. Wenn die zentrale Steuereinheit 15 der Blutbehandlungsvorrichtung das Steuersignal der Steuer- und Recheneinheit 20 empfängt, hält die zentrale Steuereinheit 15 die Blutpumpe 9 sofort an und schließt sofort die Schlauchklemme 18, so dass Blut nicht in die Umgebung gelangen kann.

Bei einer alternativen Ausführungsform werden zur Feststellung einer Verringerung der Hämoglobinkonzentration HB in Folge eines nicht ordnungsgemäßen Gefäßzugangs die an zwei aufeinanderfolgenden Zeitpunkten gemessenen Hämoglobinkonzentrationen miteinander verglichen. Die Mittel 20A der Steuer- und Recheneinheit 20 vergleichen die zu einem ersten vorausgehenden Zeitpunkt $t_1$ gemessene erste Hämoglobinkonzentration $HB_{t1}$ mit einer zu einem zweiten nachfolgenden Zeitpunkt $t_2$ gemessenen zweiten Hämoglobinkonzentration $HB_{t2}$. Wenn der Betrag der Differenz zwischen der ersten Hämoglobinkonzentration $HB_{t1}$ und der zweiten Hämoglobinkonzentration $HB_{t2}$ größer als ein vorgegebener Grenzwert ist, erzeugt die Steuer- und Recheneinheit 20 das Steuersignal, so dass die zentrale Steuereinheit 15 der Dialysevorrichtung als Eingriff in die Maschinensteuerung die Blutpumpe 9 anhält und die venöse Schlauchklemme 18 schließt. Bei dem alternativen Ausführungsbeispiel ist es grundsätzlich nicht erforderlich, mehrere Grenzwerte vorzugeben, da auch bei einer Änderung der Hämoglobinkonzentration über den gesamten Behandlungszeitraum eine plötzliche Verringerung der Hämoglobinkonzentration in Folge des Herausrutschens der venösen Punktionskanüle 8 durch den Vergleich der Hämoglobinkonzentration zu einem Zeitpunkt $t_1$ vor dem Herausrutschen der venösen Kanüle und der Hämoglobinkonzentration $HB_{t2}$ zu einem Zeitpunkt $t_2$ nach dem Herausrutschen der venösen Kanüle sicher erkannt werden kann.

Ein weiteres Ausführungsbeispiel sieht zur Erhöhung der Zuverlässigkeit der Messung noch die Überwachung der kardiopulmonalen Rezirkulation vor.

Im normalen Dialysebetrieb mit fehlerfreiem Patientenzugang gelangt laufend ein kleiner Teilstrom des zum Patienten zurückgeführten Bluts, das gereinigt und "eingedickt" ist, nicht in die Kapillarsysteme des Patienten, sondern aufgrund der kardiopulmonalen Rezirkulation und der möglichen Fistelrezirkulation zusammen mit unbehandeltem Blut über die Fistel erneut in die arterielle Blutleitung. Bei einer venösen Nadeldiskonnektion kommt deshalb kein "eingedicktes" Blut mehr auf die arterielle Seite, sondern nur noch das unbehandelte Blut aus dem Patienten. Deshalb kann in der arteriellen Schlauchleitung insgesamt ein Konzentrationsabfall gemessen werden. Zur weiteren Erhöhung der Zuverlässigkeit der erfindungsgemäßen Messung zur Erkennung einer venösen Nadeldiskonnektion kann mit einer Messung überprüft werden, ob die kardiopulmonale Rezirkulation tatsächlich in dem erwarteten Wertebereich liegt.

Die kardiopulmonale Rezirkulation wird mit der Messeinrichtung 26 gemessen. Die Steuer- und Recheneinheit 26 vergleicht die mit der Messeinrichtung 26 gemessene kardiopulmonale Rezirkulation mit einem vorgegebenen oberen und unteren Grenzwert. Bei einer Verringerung der Hämoglobinkonzentration um einen Betrag, der einen vorbestimmten Betrag überschreitet, erzeugt die Steuer- und Recheneinheit bei dieser Ausführungsform nur dann ein Steuersignal, wenn die gemessene kardiopulmonale Rezirkulation zwischen dem vorgegebenen oberen und unteren Grenzwert liegt.

Nachfolgend wird der theoretische Hintergrund für die Verringerung der Hämoglobinkonzentration in Folge des Herausrutschens der venösen Punktionskanüle 8 im Einzelnen erläutert.

Das Herausrutschen der venösen Punktionskanüle 8 führt zu einer Unterbrechung des extrakorporalen Blutkreislaufs I, so dass die Beimischung von Blut mit erhöhter Hämoglobinkonzentration über die Wege der kardiopulmonalen Rezirkulation und möglichen Fistelrezirkulation in die arterielle Blutleitung wegfällt. Dies verursacht eine Abnahme der Hämoglobinkonzentration ($\Delta HB$) in der arteriellen Blutleitung 6, die mit der erfindungsgemäßen Überwachungsvorrichtung B erfasst wird.

Der Hämatokrit an der arteriellen Punktionskanüle 8 berechnet sich aus

$$Hct_A = Hct \cdot (1-R) + Hct_V \cdot R \tag{1}$$

mit $Hct_V = Hct_A/(1-\alpha)$ und $\alpha = Q_{UF} / Q_B$ ergibt sich

$$Hct_A = Hct \cdot (1-R) \cdot \frac{1-\alpha}{1-\alpha-R} \tag{2}$$

Nach einer Umformung und Einsetzen von $R = R_A + R_{cp}$ ergibt sich

$$\frac{Hct_A - Hct}{Hct} = \frac{\alpha \cdot (R_A + R_{CP})}{1-\alpha-(R_A + R_{CP})} \tag{3}$$

Formelzeichen:

| | |
|---|---|
| $Q_B$: | Blutfluss |
| $Q_{UF}$: | Ultrafiltrationsrate |
| $R$: | gesamte Rezirkulation |
| $R_A$: | Rezirkulation in der Fistel |
| $R_{cp}$: | kardiopulmonaler Rezirkulationsanteil im extrakorporalen Blutkreislauf |
| $Hct$: | Hämatokrit deines Dialysepatienten |
| $Hct_A$: | Hämatokrit an der arteriellen Nadel |
| $Hct_v$: | Hämatokrit an der venösen Nadel |
| $HB$ | Hämoglobin Konzentration im Blut |

Die Gleichung (3) stellt durch den Wegfall der Beimischung von Blut mit erhöhter Hämoglobinkonzentration über die Wege der kardiopulmonalen Rezirkulation und der möglichen Fistelrezirkulation in die arterielle Blutleitung bedingte Änderung des Hämatokrits ($\Delta$Hct) dar. Wie signifikant die Änderung ist, lässt sich durch folgende Rechenbeispiele zeigen.

Nachfolgend wird gezeigt, dass ein Herausrutschen der venösen Punktionskanüle eine signifikante Abnahme der Hämoglobinkonzentration $\Delta$HB zur Folge hat. In dem Rechenbeispiel wird angenommen, dass die Rezirkulation $R_A$ in der Fistel = 2 % und der kardiopulmonale Rezirkulationsanteil $R_{cp}$ = 10 % ist. Für die unterschiedlichen Blutflussraten $Q_B$ von 200 ml/min und 300 ml/min und die unterschiedlichen Ultrafiltrationsraten von 500 bis 4000 ml/h ergeben sich die aus der nachfolgenden Tabelle ersichtlichen Werte:

| $Q_B$ (ml/min) | $Q_{UF}$ (ml/h) | $R_{cp}$ | $R_A$ | $\Delta Hct / Hct$ | $\Delta$HB (g/dl) bei Hct = 35% |
|---|---|---|---|---|---|
| | 500 | 0,1 | 0,02 | 0,5964 % | 0,0696 |
| | 1000 | 0,1 | 0,02 | 1,2552 % | 0,1464 |
| 200 | 2000 | 0,1 | 0,02 | 2,8038 % | 0,3271 |
| | 4000 | 0,1 | 0,02 | 7,3170 % | 0,8537 |
| | 500 | 0,1 | 0,02 | 0,3912 % | 0,0456 |
| | 1000 | 0,1 | 0,02 | 0,8097 % | 0,0943 |
| 300 | 2000 | 0,1 | 0,02 | 1,7341 % | 0,2023 |
| | 4000 | 0,1 | 0,02 | 4,0540 % | 0,4730 |

In der obigen Tabelle wird für die Umrechnung von dem Hämatokrit in die Hämoglobinkonzentration die empirische Gleichung $\Delta$HB [*in g/dl*]=[$\Delta Hct$ *in* %] / 3 verwendet, bei der es sich um eine Zahlenwertgleichung ("zugeschnittene Größengleichung") handelt.

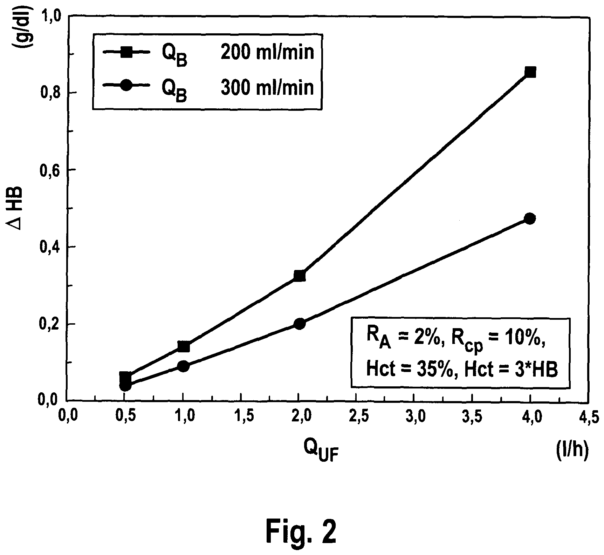

Die obige Tabelle und die Fig. 2 zeigen für zwei ausgewählte konstante Blutflussraten $Q_B$ = 200 ml/min. (oberer Graph) bzw. $Q_B$ = 300 ml/min. (unterer Graph) den Betrag $\Delta$HB der Abnahme der Hämoglobinkonzentration HB in der arteriellen Blutleitung infolge einer venösen Nadeldiskonnektion in Abhängigkeit von der Ultrafiltrationsrate $Q_{UF}$, wobei der Betrag $\Delta$HB der Abnahme der Hämoglobinkonzentration mit zunehmender Ultrafiltrationsrate größer wird, weil die "Eindickung" des gereinigten Bluts mit zunehmender Ultrafiltrationsrate zunimmt.

**Patentansprüche**

1. Vorrichtung zur extrakorporalen Blutbehandlung mit einem extrakorporalen Blutkreislauf, der eine arterielle Blutleitung mit einem arteriellen Patientenanschluss und eine venöse Blutleitung mit einem venösen Patientenanschluss aufweist, wobei in der arteriellen oder venösen Blutleitung eine Blutpumpe zum Fördern von Blut im extrakorporalen Blutkreislauf angeordnet ist, und mit einer Vorrichtung zur Überwachung des Gefäßzugangs, wobei die Vorrichtung (B) zur Überwachung des Gefäßzugangs aufweist:

Mittel (21), die derart ausgebildet sind, dass eine charakteristische Eigenschaft des in der arteriellen Blutleitung des extrakorporalen Blutkreislaufs strömenden Bluts gemessen wird,

**gekennzeichnet durch**

eine Steuer- und Recheneinheit (20), die derart ausgebildet ist, dass bei einer Veränderung der charakteristischen Eigenschaft des in der arteriellen Blutleitung strömenden Bluts um einen Betrag, der einen vorbestimmten Betrag überschreitet, auf einen nicht ordnungsgemäßen Gefäßzugang geschlossen wird, wobei die charakteristische Eigenschaft des Bluts die Hämoglobinkonzentration in dem im extrakorporalen Blutkreislauf strömenden Blut ist, wobei die Steuer- und Recheneinheit derart ausgebildet ist, dass bei einer Verringerung der Hämoglobinkonzentration des in der arteriellen Blutleitung strömenden Bluts um einen Betrag, der einen vorbestimmten Betrag überschreitet, auf einen nicht ordnungsgemäßen Gefäßzugang geschlossen wird, wobei

die extrakorporale Blutbehandlungsvorrichtung eine zentrale Steuereinheit (15) aufweist, die derart ausgebildet ist, dass die Steuereinheit einen Eingriff in die Maschinensteuerung vornimmt, wenn die Steuer- und Recheneinheit (20) der Vorrichtung (B) zur Überwachung des Gefäßzugangs einen nicht ordnungsgemäßen Gefäßzugang feststellt, wobei

die Steuereinheit (15) der Blutbehandlungsvorrichtung (A) derart ausgebildet ist, dass als Eingriff in die Maschinensteuerung eine im extrakorporalen Blutkreislauf (I) angeordnete Blutpumpe (9) angehalten wird, und/oder

die Steuereinheit (15) der Blutbehandlungsvorrichtung (A) derart ausgebildet ist, dass als Eingriff in die Maschinensteuerung ein Absperrorgan (18) zum Absperren der venösen Blutleitung (7) der Blutbehandlungsvorrichtung geschlossen wird.

2. Vorrichtung nach Anspruch 1, **dadurch gekennzeichnet, dass** die Steuer- und Recheneinheit (20) Mittel (20A) zum Vergleichen der gemessenen Hämoglobinkonzentration mit einem vorgegebenen Grenzwert aufweist, und Mittel (20B) zum Erzeugen eines Steuersignals aufweist, wenn der Betrag der Differenz zwischen der gemessenen Hämoglobinkonzentration und dem vorgegebenen Grenzwert größer als Null ist.

3. Vorrichtung nach Anspruch 1, **dadurch gekennzeichnet, dass** die Steuer- und Recheneinheit (20) Mittel (20A) zum Vergleichen der zu einem ersten vorausgehenden Zeitpunkt gemessenen ersten Hämoglobinkonzentration mit einer zu einem zweiten nachfolgenden Zeitpunkt gemessenen zweiten Hämoglobinkonzentration aufweist, und Mittel (20B) zum Erzeugen eines Steuersignals aufweist, wenn der Betrag der Differenz zwischen der ersten Hämoglobinkonzentration und der zweiten Hämoglobinkonzentration größer als ein vorgegebener Grenzwert ist.

4. Vorrichtung nach einem der Ansprüche 1 bis 3, **dadurch gekennzeichnet, dass** die Vorrichtung zur Überwachung des Gefäßzugangs Mittel (26) zum Messen der kardiopulmonalen Rezirkulation aufweist, wobei die Steuer- und Recheneinheit (20) Mittel zum Vergleichen der gemessenen kardiopulmonalen Rezirkulation mit einem vorgegebenen oberen und unteren Grenzwert aufweist, wobei die Steuer-und Recheneinheit (20) derart ausgebildet ist, dass bei einer Verringerung der Hämoglobinkonzentration um einen Betrag, der einen vorbestimmten Betrag überschreitet, auf einen nicht ordnungsgemäßen Gefäßzugang geschlossen wird, wenn die gemessene kardiopulmonale Rezirkulation zwischen dem vorgegebenen oberen und unteren Grenzwert liegt.

5. Vorrichtung nach einem der Ansprüche 1 bis 4, **dadurch gekennzeichnet, dass** die Vorrichtung zur Überwachung des Gefäßzugangs eine Alarmeinheit (24) aufweist, die einen akustischen und/oder optischen und/oder taktilen Alarm gibt, wenn die Steuer- und Recheneinheit (20) einen nicht ordnungsgemäßen Gefäßzugang feststellt.

6. Vorrichtung nach einem der Ansprüche 1 bis 5, **dadurch gekennzeichnet, dass** die Mittel (21) zum Messen der Hämoglobinkonzentration Mittel zur nicht invasiven Messung der Hämoglobinkonzentration in dem in der arteriellen Blutleitung strömenden Blut sind.

**Claims**

1. An apparatus for extracorporeal blood treatment with an extracorporeal blood circuit, which comprises an arterial blood line with an arterial patient connection and a venous blood line with a venous patient connection, wherein a blood pump for conveying blood in the extracorporeal blood circuit is disposed in the arterial or venous blood line, and which comprises a device for monitoring the vascular access, wherein the device (B) for monitoring the vascular access comprises:

means (21) which are designed such that a characteristic property of the blood flowing in the arterial blood line of the extracorporeal blood circuit is measured,
**characterised by**
a control and computing unit (20) which is designed such that, in the event of a change in the characteristic property of the blood flowing in the arterial blood line by an amount which exceeds a preset amount, it is concluded that there is an incorrect vascular access, wherein the characteristic property of the blood is the haemoglobin concentration in the blood flowing in the extracorporeal blood circuit, the control and computing unit being designed such that, in the event of a reduction in the haemoglobin concentration of the blood flowing in the arterial blood line by an amount which exceeds a preset amount, it is concluded that there is an incorrect vascular access, wherein

the extracorporeal blood treatment apparatus comprises a central control unit (15), which is designed such that the control unit performs an intervention into the machine control when the control and computing unit (20) of the device (B) for monitoring the vascular access ascertains an incorrect vascular access, wherein the control unit (15) of the blood treatment apparatus (A) is designed such that the blood pump (9) disposed in the extracorporeal blood circuit (I) is stopped as an intervention into the machine control, and/or in that the control unit (15) of the blood treatment apparatus (A) is designed such that a shut-off element (18) for shutting off the venous blood line (7) of the blood treatment apparatus is closed as an intervention into the machine control.

2. The apparatus according to claim 1, **characterised in that** the control and computing unit (20) comprises means (20A) for comparing the measured haemoglobin concentration with a preset threshold value, and means (20B) for generating a control signal when the amount of the difference between the measured haemoglobin concentration and the preset threshold value is greater than zero.

3. The apparatus according to claim 1, **characterised in that** the control and computing unit (20) comprises means (20A) for comparing the first haemoglobin concentration measured at a first preceding time with a second haemoglobin concentration measured at a second subsequent time, and means (20B) for generating a control signal when the amount of the difference between the first haemoglobin concentration and the second haemoglobin concentration is greater than a preset threshold value.

4. The apparatus according to any one of claims 1 to 3, **characterised in that** the device for monitoring the vascular access comprises means (26) for measuring the cardiopulmonary recirculation, the control and computing unit (20) comprising means for comparing the measured cardiopulmonary recirculation with a preset upper and lower threshold value, wherein the control and computing unit (20) is designed such that, in the event of a reduction in the haemoglobin concentration by an amount which exceeds a preset amount, it is concluded that there is an incorrect vascular access when the measured cardiopulmonary recirculation lies between the preset upper and lower threshold value.

5. The apparatus according to any one of claims 1 to 4, **characterised in that** the device for monitoring the vascular access comprises an alarm unit (24), which emits an acoustic and/or optical and/or tactile alarm when the control and computing unit (20) ascertains an incorrect vascular access.

6. The apparatus according to any one of claims 1 to 5, **characterised in that** the means (21) for measuring the haemoglobin concentration are means for the non-invasive measurement of the haemoglobin concentration in the blood flowing in the arterial blood line.

## Revendications

1. Dispositif de traitement de sang extracorporel avec un circuit sanguin extracorporel qui comprend une ligne de sang artérielle avec un raccordement artériel au patient et une ligne de sang veineuse avec un raccordement veineux au patient, une pompe à sang pour transporter du sang dans le circuit sanguin extracorporel étant agencée dans la ligne de sang artérielle ou veineuse, et avec un dispositif de surveillance de l'accès vasculaire, dans lequel le dispositif (B) de surveillance de l'accès vasculaire comprend :

des moyens (21) formés de telle sorte qu'une propriété caractéristique du sang s'écoulant dans la ligne de sang artérielle du circuit sanguin extracorporel est mesurée,

**caractérisé par**

une unité de commande et de calcul (20) formée de telle sorte que, en cas de modification de la propriété caractéristique du sang s'écoulant dans la ligne de sang artérielle d'une valeur dépassant une valeur prédéterminée, il est conclu à un accès vasculaire incorrect, la propriété caractéristique du sang étant la concentration en hémoglobine dans le sang s'écoulant dans le circuit sanguin extracorporel, l'unité de commande et de calcul étant formée de telle sorte que, en cas de diminution de la concentration en hémoglobine du sang s'écoulant dans la ligne de sang artérielle d'une valeur dépassant une valeur prédéterminée, il est conclu à un accès vasculaire incorrect, le dispositif de traitement de sang extracorporel comprenant une unité centrale de commande (15) formée de telle sorte que l'unité de commande procède à une intervention dans la commande machine si l'unité de commande et de calcul (20) du dispositif (B) de surveillance de l'accès vasculaire identifie un accès vasculaire incorrect,

l'unité de commande (15) du dispositif de traitement de sang (A) est formée de telle sorte que, à titre d'intervention dans la commande machine, la pompe à sang (9) disposée dans le circuit sanguin extracorporel (I) est arrêtée, et/ou l'unité de commande (15) du dispositif de traitement de sang (A) est formée de telle sorte que, à titre d'intervention dans la commande machine, un organe d'obturation (18) pour obturer la ligne de sang veineuse (7) du dispositif de traitement de sang est fermé.

2. Dispositif selon la revendication 1, **caractérisé en ce que** l'unité de commande et de calcul (20) comprend des moyens (20A) pour comparer la concentration en hémoglobine mesurée avec une valeur limite prédéfinie, et des moyens (20B) pour générer un signal de commande si la valeur de la différence entre la concentration en hémoglobine mesurée et la valeur limite prédéfinie est supérieure à zéro.

3. Dispositif selon la revendication 1, **caractérisé en ce que** l'unité de commande et de calcul (20) comprend des moyens (20A) pour comparer la première concentration en hémoglobine mesurée à un premier instant précédent avec une seconde concentration en hémoglobine mesurée à un second instant suivant, et des moyens (20B) pour générer un signal de commande si la valeur de la différence entre la première concentration en hémoglobine et la seconde concentration en hémoglobine est supérieure à une valeur limite prédéfinie.

4. Dispositif selon l'une des revendications 1 à 3, **caractérisé en ce que** le dispositif de surveillance de l'accès vasculaire comprend des moyens (26) pour mesurer la recirculation cardio-pulmonaire, l'unité de commande et de calcul (20) comprenant des moyens pour comparer la recirculation cardio-pulmonaire mesurée avec des valeurs limites supérieure et inférieure prédéfinies, l'unité de commande et de calcul (20) étant formée de telle sorte que, en cas de diminution de la concentration en hémoglobine d'une valeur dépassant une valeur prédéterminée, il est conclu à un accès vasculaire incorrect si la recirculation cardio-pulmonaire mesurée se situe entre les valeurs limites supérieure et inférieure prédéfinies.

5. Dispositif selon l'une des revendications 1 à 4, **caractérisé en ce que** le dispositif de surveillance de l'accès vasculaire comprend une unité d'alarme (24) qui émet une alarme acoustique et/ou optique et/ou tactile lorsque l'unité de commande et de calcul (20) identifie un accès vasculaire incorrect.

6. Dispositif selon l'une des revendications 1 à 5, **caractérisé en ce que** les moyens (21) pour mesurer la concentration en hémoglobine sont des moyens de mesure non invasive de la concentration en hémoglobine dans le sang s'écoulant dans la ligne de sang artérielle.

**Fig. 1**

**Fig. 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9929356 A1
- US 20040254513 A1
- WO 2006008866 A1
- US 20050038325 A1
- WO 2008000433 A1
- WO 2009065611 A1
- US 20020062098 A
- EP 1748292 A1

**In der Beschreibung aufgeführte Nicht-Patentliteratur**

- **KRÄMER ; POLASCHEGG.** Automatische Messung der Rezirkulation. *EDTNA-ERCA Journal,* 1993, vol. XIX (3